(19) **Europäisches Patentamt / European Patent Office / Office européen des brevets**

(11) **EP 4 410 116 A1**

(12) **EUROPEAN PATENT APPLICATION**
published in accordance with Art. 153(4) EPC

(43) Date of publication:
**07.08.2024 Bulletin 2024/32**

(21) Application number: **22876543.4**

(22) Date of filing: **30.09.2022**

(51) International Patent Classification (IPC):
**A23L 33/105** (2016.01)    **A23G 1/06** (2006.01)
**A23G 1/32** (2006.01)    **A23G 1/36** (2006.01)
**A23G 1/48** (2006.01)

(52) Cooperative Patent Classification (CPC):
**A23G 1/06; A23G 1/32; A23G 1/36; A23G 1/48; A23L 33/105**

(86) International application number:
**PCT/JP2022/036734**

(87) International publication number:
**WO 2023/054681 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 30.09.2021  JP 2021162427
              25.03.2022  JP 2022050372

(71) Applicant: **MEIJI CO., LTD**
**Chuo-ku**
**Tokyo 104-8306 (JP)**

(72) Inventors:
• **BABA, Kento**
  **Hachioji-shi, Tokyo 192-0919 (JP)**
• **MIYAZAKI, Megumi**
  **Hachioji-shi, Tokyo 192-0919 (JP)**
• **MATSUDA, Koki**
  **Hachioji-shi, Tokyo 192-0919 (JP)**
• **NISHIYAMA, Yuri**
  **Hachioji-shi, Tokyo 192-0919 (JP)**
• **YOSHIDA, Haruna**
  **Hachioji-shi, Tokyo 192-0919 (JP)**

(74) Representative: **TBK**
**Bavariaring 4-6**
**80336 München (DE)**

(54) **CACAO BEAN-DERIVED COMPOSITION**

(57)    To obtain a fraction derived from cacao beans containing a large amount of GABA.

A cacao bean-derived composition having a specific gravity of 1.2 or more and containing 0.55 mg/g or more of γ-aminobutyric acid (GABA) is provided.

**EP 4 410 116 A1**

**Description**

TECHNICAL FIELD

The present invention relates to a composition obtained from cacao beans. The present invention is useful in the field of food production and the like.

BACKGROUND ART

$\gamma$-Aminobutyric acid (GABA) has functionality such as improvement of blood pressure, stress relaxation, reduction of fatigue, improvement of sleep, improvement of skin elasticity, and improvement of cognitive function according to the intake amount. Therefore, it has been studied to blend GABA into various foods.

For example, Patent Document 1 describes a functional food characterized by containing GABA and polyphenol. In addition, Patent Document 2 describes a food for improving concentration characterized by containing GABA and cacao polyphenol and containing 0.05 mass% or more of the GABA. Furthermore, Patent Document 3 describes a food or beverage having a relaxing effect characterized by containing GABA as an active ingredient and containing cacao or a raw material derived from cacao. Non-Patent Document 1 describes that cacao beans contain GABA.

Recently, attention has also been paid to the taste quality-improving effect of GABA. For example, Patent Document 4 describes a method for improving the taste quality of a food or beverage in which GABA is added to a food or beverage containing at least cacao mass, cocoa cake, or cocoa powder in an amount of 0.05% or more with respect to the total mass of the food or beverage containing at least cacao mass, cocoa cake, or cocoa powder.

On the other hand, a ceramide, which is a kind of sphingolipid, is a main component of intercellular lipids present in the horny layer of the epidermis, and has a skin barrier function and a skin moisturizing function. It has been studied to incorporate a ceramide into foods with an expectation of a moisturizing effect on the skin by oral intake.

For example, Patent Document 5 describes a food containing a sugar ceramide and a diacylglycerol, and also describes that the food is for improving a skin barrier function and for moisturizing the skin. Patent Document 6 describes a glycosphingolipid-containing cocoa extract substantially free of cacao polyphenol.

On the other hand, it is known that cacao beans contain fat. Non-Patent Document 2 describes that the fat in the germ (embryo bud) and shell (exoderm) of a cacao bean contains a larger amount of unsaturated fatty acids than those of the nibs (endosperm).

PRIOR ART REFERENCES

Patent Documents

Patent Document 1: JP 2009-201383 A
Patent Document 2: JP 2005-13127 A (JP 4041020 B2)
Patent Document 3: JP 2005-348656 A
Patent Document 4: JP 2007-6853 A
Patent Document 5: JP 2005-295995 A (JP 4359205 B2)
Patent Document 6: US 2001/0041683 A1

Non-Patent Documents

Non-Patent Document 1: Angela Marseglia, Gerardo Palla, Augusta Caligiani (2014) Presence and variation of $\gamma$-aminobutyric acid and other free amino acids in cocoa beans from different geographical origins. Food Research International 63, 360-366
Non-Patent Document 2: Yoshitsugu Nakanishi, 1970, Cacao Butter and Substituted Cacao Butter, Oil Chemistry, Vol. 19(8): 722-733

SUMMARY OF THE INVENTION

Object to be Achieved by the Invention

The nib (endosperm), the edible part of cacao beans, is known to contain GABA, but in relatively small amount. Therefore, in conventional foods using cacao beans as a raw material, such as chocolate, it cannot be expected that a large amount of GABA derived from cacao beans is contained, and GABA as a functional component is externally added.
Conventional processed products of cacao beans include cocoa butter, cacao mass, and cocoa powder. GABA is not contained in cocoa butter because of its water-solubility, and the content of GABA in cacao mass, which is obtained by grinding nibs, is not large. On the other hand, cocoa powder is produced by squeezing cacao mass to remove a part of cocoa butter, and therefore it is expected that the content of GABA is relatively high. However, it is difficult to take a large amount of cocoa powder because of its strong bitterness.
Furthermore, though glucosylceramides are widely contained in plants, it has not been known how much and in what fraction glucosylceramides are contained in cacao beans. Therefore, it is unknown what fraction should be actively ingested in order to obtain sphingolipids such as glucosylceramides from cacao beans.

Means for Achieving the Object

The present inventors have intensively studied the functionality of cacao beans. As a result, it has been found that cacao beans contain a large amount of GABA in a fraction having a certain specific gravity. It has also been found that this fraction contains characteristic triglycerides and that this fraction has a unique formulation of fatty acids. In addition, knowledge about the amount and localization of sphingolipids contained in cacao beans was also obtained. Based on these findings, the present invention has been completed. The present application provides the following inventions.

[1] A cacao bean-derived composition having a specific gravity of 1.2 or more and containing 0.55 mg/g or more of γ-aminobutyric acid (GABA).
[2] A cacao bean-derived composition containing at least one triglyceride selected from the group consisting of LLL, LLO, PLL, and OLO; and 0.55 mg/g or more of GABA, wherein L represents a linoleic acid residue, O represents an oleic acid residue, and P represents a palmitic acid residue.
[3] The composition according to 1 or 2, wherein total triglycerides in the composition contain LLL, LLO, PLL, and OLO in a sum total amount of 1% or more.
[4] The composition according to 1 or 2, wherein total triglycerides in the composition contain LLL, LLO, PLL, OLO, PLO, and PLP in a sum total amount of 5% or more.
[5] The composition according to 1 or 2, wherein total triglycerides in the composition contain PLO and PLP in a sum total amount of 5% or more.
[6] The composition according to any one of 1 to 5, wherein total triglycerides in the composition contain at least one selected from the group consisting of SOP and SOS in an amount of less than 20%, wherein S represents a stearic acid residue.
[7] A cacao bean-derived composition,

having a fatty acid formulation which comprises 1) to 4):

1) total fatty acids in the composition contain palmitic acid, stearic acid, and oleic acid in a sum total amount of less than 90%;
2) total fatty acids in the composition contain linoleic acid in an amount of 20 to 40%;
3) total fatty acids in the composition contain capric acid, lauric acid, myristic acid, and pentadecanoic acid in a sum total amount of 0.1 to 5%; and
4) total fatty acids in the composition contain palmitic acid in an amount of 20% or more, and

containing 0.55 mg/g or more of GABA.

[8] A food containing the composition according to any one of 1 to 7.
[9] A processed product of the composition according to any one of 1 to 7.
[10] A cacao bean-derived composition having a specific gravity of 1.2 or more and containing 0.26 mg/g or more of a sphingolipid.
[11] A cacao bean-derived composition containing at least one triglyceride selected from the group consisting of LLL, LLO, PLL, and OLO; and 0.26 mg/g or more of a sphingolipid, wherein L represents a linoleic acid residue, O represents an oleic acid residue, and P represents a palmitic acid residue.

[12] The composition according to 10 or 11, wherein total triglycerides in the composition contain LLL, LLO, PLL, and OLO in a sum total amount of 1% or more.

[13] The composition according to 10 or 11, wherein total triglycerides in the composition contain LLL, LLO, PLL, OLO, PLO, and PLP in a sum total amount of 5% or more.

[14] The composition according to 10 or 11, wherein total triglycerides in the composition contain PLO and PLP in a sum total amount of 5% or more.

[15] The composition according to any one of 10 to 14, wherein total triglycerides in the composition contain at least one selected from the group consisting of SOP and SOS in an amount of less than 20%, wherein S represents a stearic acid residue.

[16] A cacao bean-derived composition,

having a fatty acid formulation which comprises 1) to 4):

1) total fatty acids in the composition contain palmitic acid, stearic acid, and oleic acid in a sum total amount of less than 90%;
2) total fatty acids in the composition contain linoleic acid in an amount of 20 to 40%;
3) total fatty acids in the composition contain a total amount of capric acid, lauric acid, myristic acid, and pentadecanoic acid in a sum total amount of 0.1 to 5%; and
4) total fatty acids in the composition contain palmitic acid in an amount of 20% or more, and

containing 0.26 mg/g or more of a sphingolipid.

[17] A food containing the composition according to any one of 10 to 16.

[18] A processed product of the composition according to any one of 10 to 16.

[19] The composition according to any one of 10 to 18, wherein the sphingolipid is contained in an amount of 0.3 mg/g or more and 1 mg/g or less.

[20] A method for producing a composition containing at least one selected from GABA and a sphingolipid, the method comprising:
obtaining a fraction having a specific gravity of 1.2 or more from a cacao bean-ground product.

[21] A method for producing a food, the method comprising:
producing a composition by the method according to 20, and adding the produced composition to a food.

[22] A method for producing a food, the method comprising:

adding a cacao bean-ground product to a food, wherein the processed product of cacao beans is a cacao bean-ground product having a specific gravity of 1.2 or more, and
thereby a cacao-derived GABA is reinforced in the food.

[23] A method for producing a food, the method comprising:
adding a processed product of cacao beans to a food, wherein the processed product of cacao beans is a cacao bean-ground product having a specific gravity of 1.2 or more, and thereby a cacao-derived sphingolipid is reinforced in the food.

## MODES FOR CARRYING OUT THE INVENTION

[Cacao bean-derived composition]

The present invention provides a cacao bean-derived composition having a specific gravity of 1.2 or more and containing at least one selected from $\gamma$-aminobutyric acid (GABA) and a sphingolipid.

(Raw material cacao beans)

The term cacao bean refers to a seed of cacao (Theobroma cacao). The variety or production area of the cacao beans used as the raw material for the composition of the present invention are not particularly limited. Examples of cacao varieties include Forastero, Criollo, Trinitario varieties, derivatives and hybrids thereof. Examples of the production area include Ghana, Cote d'Ivoire, Nigeria, Brazil, Venezuela, and Trinidad and Tobago.

In general, cacao beans used as a raw material for chocolate are taken out from a cacao pod (cacao nut) together with pulp, and subjected to processing such as fermentation. Since GABA is a relatively heat-stable component, various raw material cacao beans can be used for the composition of the present invention regardless of whether they have been processed or not or how much they have been processed. Examples of the processing of cacao beans include fermentation, removal of pulp, drying, and roasting. A cacao bean includes an endosperm (nib), an embryo bud (germ), and an exoderm (shell).

In order to obtain a composition having a high GABA content, it is preferable that the raw material cacao beans have not undergone a roasting step at a temperature higher than 160°C. The raw material cacao beans to be preferably used in the present invention are preferably not roasted and preferably fermented for shorter period.

(Composition)

The composition of the present invention is a material processed using cacao beans as a raw material. The composition of the present invention has a certain specific gravity as described later and is typically a solid obtained by selecting one obtained by grinding cacao beans. The water content is relatively small and may be about the same as or less than that of the raw cacao bean.

(Specific gravity)

The composition of the present invention has a certain specific gravity. According to the study the present inventors made, by selecting a fraction having a certain specific gravity from a cacao beans-ground product, a composition having a high content of GABA can be made, and a composition having a high content of sphingolipid can be made.

The specific gravity is 1.0 or more, preferably 1.1 or more, more preferably 1.2 or more, still more preferably 1.3 or more, and particularly preferably 1.3 to 1.5. When the specific gravity is 1.2 or more, it is considered that a fraction containing a large amount of a desired component is collected, and a composition having a high content of at least one selected from GABA and a sphingolipid can be obtained. Examples of a cacao bean-derived composition having a specific gravity of 1.2 or more include a composition obtained by subjecting the germ to a process, such as grinding, as necessary. A composition containing the germ can be a good source of GABA and sphingolipids described below.

With regard to the present invention, unless otherwise specified, the term specific gravity refers to a specific gravity measured with a density measuring kit from Mettler-Toledo International Inc. or a measurement method based on the same principle. This measurement method utilizes the Archimedes' principle and is determined by the following formula.

[Mathematical formula 1]

$$\text{Density } \rho = \frac{A}{A-B}(\rho_0 - \rho_L) + \rho_L$$

$\rho$ = Sample density
A = Sample weight in atmosphere
B = Sample weight in replacement liquid
$\rho_0$ = Replacement liquid density
$\rho_L$ = Atmosphere density

When the specific gravity is measured, the replacement liquid may be water or ethanol, which are easy to handle. The temperature of the replacement liquid at the time of measurement can be appropriately set. The density of the replacement liquid corresponding to the temperature of the replacement liquid at the time of measurement may be used for calculation.

(GABA)

The composition of the present invention is enriched in GABA. Specifically, the content is 0.25 mg/g or more. The GABA content is preferably 0.55 mg/g or more, 0.6 mg/g or more, 0.7 mg/g or more, 0.8 mg/g or more, 0.9 mg/g or more, 1.0 mg/g or more, 1.5 mg/g or more, 2 mg/g or more, or 3 mg/g or more. The upper limit of the GABA content in the composition may be, but is not limited to, 10 mg/g or less, 9 mg/g or less, 8 mg/g or less, 7 mg/g or less, 6 mg/g or less, 5 mg/g or less, or 4 mg/g or less. These lower limit values and upper limit values each can be arbitrarily selected and combined.

The content of GABA can be appropriately measured by those skilled in the art. Methods for measuring GABA as a food component are well known to those skilled in the art. Specific examples of the measurement method include the method described in the section of Examples of the present application.

(Sphingolipid)

In the present invention, the term sphingolipid refers to a complex lipid containing sphingoids as a long-chain base component unless otherwise specified. Sphingolipids can be classified into glycosphingolipids and sphingophospholipids, and glycosphingolipids (It may also be referred to as glycosylceramide or sugar ceramide.) include glucosylceramide (those in which a glucose is bound to a ceramide). The sphingolipid derived from cacao beans may be referred to as a cacao sphingolipid (or cacao bean sphingolipid) or cacao ceramide (or cacao bean ceramide).

The composition of the present invention contains at least 0.23 mg/g or more of a sphingolipid (when a plurality of kinds of sphingolipids are contained, the total amount thereof). The content is preferably 0.26 mg/g, 0.3 mg/g or more, 0.37 mg/g or more, 0.4 mg/g or more, 0.5 mg/g or more, 0.6 mg/g or more, 0.8 mg/g or more, 1 mg/g or more, 2 mg/g or more, or 3 mg/g or more. The upper limit of the sphingolipid content in the composition may be, but is not limited to, 10 mg/g or less, 9 mg/g or less, 8 mg/g or less, 7 mg/g or less, 6 mg/g or less, 5 mg/g or less, or 4 mg/g or less. These lower limit values and upper limit values each can be arbitrarily selected and combined.

The content of sphingolipid can be measured by the method described in the section of Examples of the present specification. This method is originally a method for quantifying glucosylceramide, and can be said to be a method for analyzing sphingolipid for the cacao bean-derived composition of the present invention. Quantification can be performed by comparison with a standard (for example, rice glucosylceramide).

(Triglycerides)

The present invention also provides a cacao bean-derived composition containing at least one triglyceride selected from the group consisting of LLL, LLO, PLL, and OLO, and containing at least one selected from 0.55 mg/g or more of GABA and 0.26 mg/g or more of a sphingolipid. In the present invention, a triglyceride is represented by three abbreviations of fatty acid residue arranged in the order of α-position, β-position, and γ-position. The following abbreviations are used unless otherwise specified.

P: Palmitic acid residue, S: Stearic acid residue, O: Oleic acid residue, L: Linoleic acid residue, Ln: Linolenic acid residue, and A: Arachidic acid residue.

According to the study the present inventors made, the composition of the present invention contains LLL, LLO, and PLL as a triglyceride in which the β-position is linoleic acid. Such triglycerides were not detected from cacao nib, which is a conventional processed product of cacao beans. The composition of the present invention may contain OLO as a triglyceride in which the β-position is linoleic acid. It has been found that the OLO content is low in cacao nib, which is a conventional processed product of cacao beans.

In a preferred embodiment, the total triglycerides in the composition contain LLL, LLO and PLL in a sum total amount of 1% or more, preferably 3% or more, 5% or more, 7% or more, 9% or more, or 11% or more. The upper limit may be, but is not limited to, 23 mg/g or less, 21 mg/g or less, 18 mg/g or less, 19 mg/g or less, 17 mg/g or less, or 15 mg/g or less. These lower limit values and upper limit values each can be arbitrarily selected and combined.

Further, according to the study the present inventors made, the total amount of LLL, LLO, PLL, OLO, PLO, and PLP was relatively high in the composition of the present invention. On the other hand, the conventional cacao nib contained OLO, PLO, and PLP, but the total amount thereof was relatively low.

In a preferred embodiment, the total triglycerides in the composition contain LLL, LLO, PLL, OLO, PLO, and PLP in a sum total amount of 5% or more, preferably 7% or more, 10% or more, 15% or more, 20% or more, or 25% or more. The upper limit may be, but is not limited to, 32 mg/g or less, 31 mg/g or less, 30 mg/g or less, 29 mg/g or less, 28 mg/g or less, or 27 mg/g or less. These lower limit values and upper limit values each can be arbitrarily selected and combined.

In a preferred embodiment, the total triglycerides in the composition contain PLO and PLP in an amount of 5% or more, preferably 7% or more, 8% or more, 9% or more, 10% or more, or 11% or more. The upper limit may be, but is not limited to, 26 mg/g or less, 24 mg/g or less, 22 mg/g or less, or 20 mg/g or less. These lower limit values and upper limit values each can be arbitrarily selected and combined.

The triglyceride formulation may be analyzed by a standard method for analyzing food components, for example, a method according to the analytical manual of Standard tables of food composition in japan-2015-(seventh revised edition) and Standard methods for the analysis of fats, oils and related materials (made by Japan Oil Chemists' Society). The triglyceride formulation analysis may be performed by performing pretreatment by an acid hydrolysis method, then purifying the triglyceride by column chromatography (2.5.5-2013), and then performing high performance liquid chromatography (2.4.6.2-2013).

(Fatty acid formulation)

The present invention also provides a cacao bean-derived composition having the following fatty acid formulation which comprises 1) to 4):

1) total fatty acids in the composition contain palmitic acid, stearic acid, and oleic acid in a sum total amount of less than 90%;
2) total fatty acids in the composition contain linoleic acid in an amount of 20 to 40%;
3) total fatty acids in the composition contain capric acid, lauric acid, myristic acid, and pentadecanoic acid in a sum total amount of 0.1 to 5%; and
4) total fatty acids in the composition contain palmitic acid in an amount of 20% or more, and

at least one selected from 0.55 mg/g or more of GABA and 0.26 mg/g or more of a sphingolipid.

The fatty acid derived from cacao beans may be measured for caprylic acid, capric acid, lauric acid, myristic acid, pentadecanoic acid, palmitic acid, palmitoleic acid, heptadecanoic acid, stearic acid, oleic acid, linoleic acid, linolenic acid, arachidic acid, eicosenoic acid, behenic acid, and lignoceric acid. The total amount of the fatty acids may be the total amount of the fatty acid derived from cacao beans. This is because, when these fatty acids are measured, almost all fatty acids derived from cacao beans are measured.

According to the study the present inventors made, it has been found that the cacao bean-derived composition of the present invention having a certain specific gravity contains capric acid, lauric acid, myristic acid, and pentadecanoic acid, which are not contained in conventional cacao nib. The total amount thereof in the total fatty acids is 0.8% or more, preferably 1% or more, 1.1%, or 1.2% or more. In addition, the total fatty acids in the composition of the present invention contain in a relatively large amount of palmitic acid, for example, in an amount of 14% or more, and preferably 18% or more, 22% or more, 26% or more, or 30% or more.

Since the cacao bean-derived composition of the present invention is different from conventional cacao nib in the fatty acid formulation, it can be expected that the cacao bean-derived composition exhibits functional characteristics different from those of conventional processed products of cacao beans. In addition, the cacao bean-derived composition of the present invention can be used not only as a source of GABA or sphingolipids but also as a source of linoleic acid.

The fatty acid formulation may be analyzed by a standard method for analyzing food components, for example, a method according to the analytical manual of Standard tables of food composition in japan-2015-(seventh revised edition) and Standard methods for the analysis of fats, oils and related materials. The fatty acid analysis may be performed by a capillary gas chromatography method (see 2.2.1-2013) after performing pretreatment by an acid hydrolysis method.

[Processed product]

The composition of the present invention can be a processed product. Examples of the processed product include a powder, a granule, a compression molded solid of powder, a paste in which oils and fats or liquid is mixed, a solvent extract of the cacao bean-derived composition, and a fraction, a purified product, a concentrate, a dried product, and an extraction residue thereof.

[Food]

The composition of the present invention and the processed product thereof can be used as a raw material of food. The food using the cacao bean-derived composition or the processed product thereof as a raw material can be in any form, such as confectionery, beverage, seasoning, processed food, prepared food, and soup. More specifically, it can be chocolate products (chocolate, semi-chocolate, chocolate confectionery, semi-chocolate confectionery), gummy, tablet confectionery, chewing gum, candy, snack, biscuit, cracker, yokan, bean jam, ice cream, frozen confectionery, jelly, pudding, filling, soft drink, smoothie, fruit juice drink, vegetable drink, soy milk drink, tea drink, coffee drink, powdered drink, carbonated drink, alcoholic drink, spread, margarine, flavor seasoning, dressing, sauce, tare, tsuyu, roux, yogurt, cream, cheese, milk drink, lactic acid bacteria drink, bread, pasta, pizza crust, cake, cake mix, prepared milk powder, liquid food, food for patients, nutritional food, supplement, tablet, frozen food, retort food, canned food, microwave oven food, instant soup, instant noodle, and tofu.

The content of the composition of the present invention or the processed product thereof in the food is not particularly limited, and can be appropriately set according to the form of the food or the like. For example, the content of the composition of the present invention or the processed product thereof in the food can be 1 to 99%, may be 10 to 90%, or may be 20 to 60%.

The food containing the composition of the present invention or the processed product thereof may contain other food raw materials, other active ingredients and nutritional ingredients acceptable as a food product, in addition to the composition of the present invention or the processed product thereof. In addition, an additive acceptable as a food may be further contained.

In the production of the food, the stage of blending the composition of the present invention or the processed product thereof is not particularly limited as long as the properties of the composition of the present invention or the processed product thereof are not significantly impaired. For example, the composition or processed product thereof can be blended by mixing with other raw materials at an early stage of the production.

The food includes not only those for humans but also those for animals other than humans, unless otherwise stated. The food includes, unless otherwise stated, a general food, a functional food, a nutritional composition, a therapeutic food (a meal serving therapeutic purposes, or a meal cooked based on a menu prepared by a dietitian or the like in accordance with a dietary recipe presented by a doctor), a dietary meal, an ingredient adjustment meal, a nursing meal, and a food for treatment support. The food includes not only solid products but also liquid products such as beverage, health drink, liquid food, and soup, unless otherwise specified. The functional food refer to foods that can impart a predetermined functionality to a living body, and generally include health foods, for example, food for specified health uses (including conditional food for specified health uses [Tokuho]), food with function claims, food with health claims including food with nutrient function claims, food for special dietary uses, nutritional supplement food, health supplement food, supplement (for example, those of various kinds of dosage forms such as tablet, coated tablet, sugar-coated tablet, capsule, and liquid drug), cosmetic food (for example, diet food), and so forth. In addition, concerning the present invention, the term "functional food" also includes health foods to which a health claim based on the food standard of Codex (Joint FAO/WHO Codex Alimentarius Commission, CAC) is applied.

The food containing the composition of the present invention and the processed product thereof can be labeled with the function and the intended use (application) according to the function of the contained component, and labeled with an indication that the administration and intake of the food is recommended for a specific target. The indication can be a direct or indirect indication. Examples of the direct indication include indications on tangible objects such as the product itself, package, container, label, tag, of the product, etc. Examples of the indirect indication include advertisement or publicity on such places or by such means as website, storefront, pamphlet, exhibition, seminar such as media seminar, book, newspaper, magazine, television, radio, mail, e-mail, and voice. Examples of the intended use (application) to be labeled include "alleviating temporary mental stress associated with office work", "having a function suitable for a person with higher blood pressure ", "alleviating temporary mental stress by work or study", "enhancing skin moisturizing power (barrier function)", "adjusting skin tone", "helping to make skin less dry", "making it difficult to release moisture in the skin of the face and the body (cheek, back, elbow, instep of foot)", "helping to protect skin moisture", and the like.

[Production Method]

The present invention provides a method for producing a composition containing at least one selected from GABA and a sphingolipid. The production method of the present invention includes a step of obtaining a fraction having a specific gravity of 1.2 or more, preferably 1.3 or more, and more preferably 1.3 to 1.5 from a ground product of cacao beans. Also provided is a method for producing a food including a step of adding the produced composition, and including such a step. The step of obtaining a fraction having a specific gravity of 1.2 or more from a ground product of cacao beans can be performed using existing equipment.

The present invention also provides a method for producing a food, the method including a step of adding a processed product of cacao beans to a food, wherein the processed product of cacao beans is a ground product of cacao beans having a specific gravity of 1.2 or more, preferably 1.3 or more, and more preferably 1.3 to 1.5, and thereby at least one selected from a cacao-derived GABA (or cacao bean-derived GABA) and a cacao-derived sphingolipid (or cacao bean-derived sphingolipid) is reinforced in the food.

EXAMPLES

[Production Example 1: Cacao bean-derived composition]

A cacao bean-derived composition (parts of cacao bean-ground product, having a specific gravity of 1.2 or more) was produced by the following method.

Cacao beans are ground by a crusher, and then a ground product which passes through a shifter having a mesh size of 3.1 mm in a winnower and remains in the shifter having a mesh size of 1.0 mm is collected. From this ground product, a 1.4 to 2.0 mm fraction is size-sorted again using a vibration sieve. From this size-sorted product, a portion having a specific gravity of 1.2 or more is selected and sorted using a specific gravity sorter (yield of about 1% from raw material cacao beans). Hereinafter, the cacao bean-derived composition (parts of cacao bean-ground product, having a specific gravity of 1.2 or more) may be simply referred to as the composition.

[Production Example 2: Processed product of cacao bean-derived composition]

A water-extract (essence) of a cacao bean-derived composition was produced by the following method.

(1) In a 50 mL plastic tube, 4.5 g of the cacao bean-derived composition produced by the method of Production Example 1 is weighed.
(2) To (1), 25 mL of hot water at 85°C is added.
(3) Extraction is performed at normal temperature for 30 minutes using a shaker (150 rpm, amplitude 4 cm).
(4) After extraction, the extract is immediately cooled in ice.

[Production Example 3: Chocolate]

A sweet chocolate using a cacao bean-derived composition was produced by the following method.

(1) 53.3 g of the cacao bean-derived composition produced by the method of Production Example 1 is ground to about 200 μm with a mill.
(2) To (1), 20 g of cacao butter, 16.0 g of powdered sugar, and 0.2 g of lecithin are added and mixed, and then ground with a refiner to form flakes.
(3) After (2) is kneaded and liquefied, 10.2 g of cacao butter and 0.3 g of lecithin are added and mixed to obtain chocolate.
(2) (3) is tempered, then poured into a mold, and cooled.

A milk chocolate using a cacao bean-ground product having a specific gravity of 1.2 or more was produced by the following method.

(1) 30.8 g of the cacao bean-derived composition produced by the method of Production Example 1 is ground to about 200 μm with a mill.
(2) To (1), 12.8 g of cacao butter, 25.0 g of powdered sugar, 17.0 g of whole milk powder, and 0.5 g of lecithin are added and mixed, and then ground with a refiner to form flakes.
(3) After (2) is kneaded and liquefied, 13.9 g of cacao butter is added and mixed to obtain chocolate.
(4) (3) is tempered, then poured into a mold, and cooled.

[Production Example 4: Comparative Example]

General cacao mass, cocoa powder, and commercially available chocolate were used. The cacao mass was prepared by subjecting cacao beans to fermentation, drying, roasting, cracking, separation, and grinding steps according to conventional methods. The cocoa powder was prepared by treating the cacao mass with a conventional hydraulic press machine so as to have an oil content of 12%.
The cacao nib was prepared by subjecting cacao beans to fermentation, drying, roasting, cracking, and separation steps according to conventional methods.

[Measurement method]

<Specific gravity>

The specific gravity was measured using "Weight method, buoyancy" of a density measuring kit (Mettler-Toledo International Inc.). This measurement principle utilizes the "Archimedes' principle", which means "All objects in the fluid are subjected to buoyancy equal to the weight of the fluid displaced by the object.". Specific gravity is obtained by the following equation.

[Mathematical formula 2]

$$\text{Density } \rho = \frac{A}{A-B}(\rho_0 - \rho_L) + \rho_L$$

ρ = Sample density
A = Sample weight in atmosphere
B = Sample weight in replacement liquid

$\rho_0$ = Replacement liquid density
$\rho_L$ = Atmosphere density

In this measurement, the replacement liquid was water or ethanol. The temperature of the replacement liquid was 23.0 to 23.5°C. One sample was measured 30 times, and the average value was taken as the density of the sample.

<Method for measuring GABA>

GABA was measured by the following method.

(1) Only a sample having a high moisture content is freeze-dried and then ground with a mill. If necessary, liquid nitrogen is used during grinding.
(2) 2 g of the ground sample is weighed in a Falcon tube, added with 30 ml of hexane, and then shaken for 20 minutes.
(3) Centrifugation is performed at 4°C and 3000 rpm for 15 minutes.
(4) The supernatant is disposed.
(5) (2) to (4) are performed twice in total.
(6) The resultant is air-dried in the draft overnight.
(7) (6) is added with 25 ml of 80% ethanol and shaken for 1 hour.
(8) Centrifugation is performed at normal temperature and 3000 rpm for 10 minutes.
(9) The supernatant is collected in a 50 ml volumetric flask.
(10) The residue is added with 25 ml of 80% ethanol again and shaken for 1 hour.
(11) Centrifugation is performed at normal temperature and 3000 rpm for 10 minutes.
(12) The supernatant is recovered into the 50 ml volumetric flask in (9), and then the volume is made up.
(13) Centrifugation is performed at 25°C and 15,000 rpm for 20 minutes.
(14) The resultant is diluted 2 times with 0.02 N hydrochloric acid.
(15) The resultant is put into a vial with a filter (Pore size: 0.45 $\mu$m).
(16) Analysis is performed using Hitachi L-8800 high-speed amino acid analyzer.

Column: Packed column # 2622 for Hitachi HPLC
Mobile phase: Buffer for HITACHI High Performance Amino Acids Analyzer (PF-1, PF-2, PF-3, PF-4, PF-RG)
Reaction liquid: Amino acid kit ninhydrin test solution (identification number: for HITACHI) (FUJIFILM Wako Pure Chemical Corporation)
Flow rate: 0.2 ml/min
Column temperature: 50°C
Sample injection: 20 $\mu$L
Calibration curve range: one-point calibration curve

<Method for analyzing triglyceride and fatty acid>

Triglycerides and fatty acids were analyzed by the following method.
The analysis is performed by a method in accordance with the analytical manual of Standard tables of food composition in japan-2015-(seventh revised edition) and Standard methods for the analysis of fats, oils and related materials. The triglyceride formulation analysis is performed by performing pretreatment by an acid hydrolysis method, then purifying the triglyceride by column chromatography (2.5.5-2013), and then performing high performance liquid chromatography (2.4.6.2-2013). The fatty acid analysis is performed by a capillary gas chromatography method (see 2.2.1-2013) after performing pretreatment by an acid hydrolysis method.

<Measurement of Sphingolipid>

The sphingolipid contained in the sample was measured as a sugar ceramide by the following method.
Pretreatment for measurement

(1) In a 50 mL centrifuge tube, 0.8 g of the composition of the present invention or a dried product of the processed product of the composition of the present invention is weighed.
(2) (1) is added with 16 ml of hexane and 16 mL of hexane-saturated methanol.
(3) (2) is shaken at normal temperature for 15 minutes.
(4) (3) is centrifuged at 5°C for 15 minutes (3000 rpm) to obtain the upper layer.
(5) (4) is added with 16 mL of hexane-saturated methanol.

(6) (5) is shaken at normal temperature for 15 minutes.

(7) (6) is centrifuged at 5°C for 15 minutes (3000 rpm), and the upper layer is discarded.

(8) The lower layer of (7) is combined with the lower layer of (4), and dried and solidified under nitrogen stream.

(9) The dried solid of (8) is added with 0.4 mL of a solution of chloroform : methanol = 2 : 1, 8 mL of 0.5 M potassium hydroxide solution in methanol, and 6 mL of water.

(10) (9) is shaken at 37°C for 180 minutes.

(11) (10) is added with 16 ml of chloroform.

(12) (11) is shaken at normal temperature for 15 minutes.

(13) (12) is centrifuged at 5°C for 15 minutes (3000 rpm) to obtain the upper layer.

(14) (13) is added with 16 ml of chloroform.

(15) (14) is shaken at normal temperature for 15 minutes.

(16) (15) is centrifuged at 5°C for 15 minutes (3000 rpm), and the upper layer is discarded.

(17) The lower layer of (16) is combined with the lower layer of (13), and dried and solidified under nitrogen stream.

(18) (17) is added with 12.8 mL of chloroform, 6.4 mL of methanol, and 4.8 mL of water.

(19) (18) is shaken at normal temperature for 15 minutes.

(20) (19) is centrifuged at 5°C for 15 minutes (3000 rpm), and the upper layer is discarded.

(21) The lower layer of (20) is added with 6.4 mL of methanol and 4.8 mL of water.

(22) (21) is shaken at normal temperature for 15 minutes.

(23) (22) is centrifuged at 5°C for 15 minutes (3000 rpm), and the upper layer is discarded.

(24) The lower layer of (23) is dried and solidified under nitrogen stream.

(25) (24) is added with 2 mL of a solution of chloroform : methanol = 2 : 1.

(26) (25) is stirred with a vortex mixer and filtered through a membrane filter (0.2 $\mu$m).

(27) (26) is analyzed using Agilent 1200 series HPLC.

Measurement method

Measurement is performed under the following conditions.

Column: Inertsil SIL-100A 5 $\mu$m 4.6 x 150 mm
Mobile phase A: Hexane : IPA = 100 : 1 Mobile phase B: Methanol: IPA =4: 6

[Table 1]

| Gradient | |
|---|---|
| Time (min) | %B |
| 0 | 4 |
| 20 | 25 |
| 23 | 25 |
| 24 | 100 |
| 28 | 100 |
| 29 | 4 |
| 39 | 4 |

Detector: Agilent 385-ELSD Neb 40°C Eva 70°C GasFlow 1.0 SLM
Flow rate: 1 ml/min
Column temperature: 40°C
Sample injection: 20 $\mu$L
Calibration curve range: three-point calibration curve

For quantification, a rice ceramide (Nagara Science, Glucosylceramide, from Rice (Glucosylceramide mix.)) is used as a standard, and the value is calculated.

[Measurement results]

<GABA content and sphingolipid content>

The measurement results of GABA and sphingolipid are shown in the following table.

[Table 2]

| Sample number | Conditions | | | GABA (mg/g) | | Sphingolipid (mg/g) | |
|---|---|---|---|---|---|---|---|
| | Production area | Fermentation | Roast | Cacao bean-derived composition | Cacao nib | Cacao bean-derived composition | Cacao nib |
| 1 | Asia | Unfermented | Unroasted | 3.46 | 0.61 | - | - |
| 2 | Asia | Unfermented | Unroasted | 3.71 | 0.70 | - | - |
| 3 | South America | Unfermented | Unroasted | 3.31 | 0.74 | 0.49, 0.49 | 0.12 |
| 4 | South America | Unfermented | Unroasted | 1.39 | 0.49 | 0.39 | 0.17 |
| 5 | South America | Fermented | Unroasted | 1.34 | 0.63 | 0.36, 0.54 | 0.16, 0.21 |
| 6 | South America | Fermented | Unroasted | 0.89 | 0.40 | - | - |
| 7 | South America | Fermented | Roasted | 0.91 | 0.34 | 0.38 | 0.25 |
| 8 | South America | Fermented | Roasted | 0.66 | 0.32 | - | - |
| 9 | South America | Fermented | Roasted | 0.62 | 0.28 | 0.38 | - |
| 13 | Africa | Fermented | Unroasted | 0.93 | 0.35 | 0.37, 0.32 | 0.22 |
| 14 | South America | Fermented | Unroasted | 1.00 | 0.42 | 0.32 | - |
| 15 | South America | Fermented | Unroasted | 1.11 | 0.43 | 0.27 | - |
| 16 | South America | Fermented | Unroasted | 0.96 | 0.44 | 0.36 | - |
| 17 | South America | Fermented | Unroasted | 1.22 | 0.57 | 0.36 | - |
| 18 | South America | Fermented | Unroasted | 1.49 | 0.53 | 0.34 | - |
| 19 | South America | Fermented | Unroasted | 1.28 | 0.53 | - | - |
| 20 | South America | Fermented | Unroasted | 1.63 | 0.72 | 0.28 | - |
| 21 | South America | Fermented | Unroasted | 1.02 | 0.42 | - | - |
| 22 | Africa | Fermented | Roasted | 1.07 | 0.38 | - | - |

(continued)

| Sample number | Conditions | | | GABA (mg/g) | | Sphingolipid (mg/g) | |
|---|---|---|---|---|---|---|---|
| | Production area | Fermentation | Roast | Cacao bean-derived composition | Cacao nib | Cacao bean-derived composition | Cacao nib |
| 24 | South America | Fermented | Roasted | 0.74 | 0.39 | - | - |
| 25 | South America | Fermented | Roasted | 0.93 | 0.42 | - | - |
| 26 | South America | Fermented | Roasted | 0.56 | - | - | - |
| 27 | Africa | Fermented | Roasted | 1.19 | - | 0.23 | - |

All of the cacao bean-derived compositions obtained by the above-described method contained 0.55 mg/g or more of GABA. All of the cacao bean-derived compositions obtained by the above-described method contained 0.23 mg/g or more of a sphingolipid.

The moisture content was as follows.

Composition of Sample 22: 6.18%
Dry nib of Sample 22: 2.53%
Composition of Sample 13: 6.87%
Raw nib of Sample 13: 3.61%

<Comparison of GABA with conventional product and the like>

The measurement results of the GABA content in the general cacao mass, cocoa powder, and commercially available chocolate, and the GABA content in the essence of Production Example 2 and the chocolate of Production Example 3 are shown in the following table.

[Table 3]

| Sample | GABA (mg/g) |
|---|---|
| Cacao mass (cacao beans under the conditions of Sample 21 are used) | 0.39 |
| Cocoa powder (cacao beans under the conditions of Sample 27 are used) | 0.76 |
| Meiji Milk Chocolate (manufactured by Meiji Co., Ltd.) | 0.08 |
| Meiji Chocolate Kouka cacao 72% (manufactured by Meiji Co., Ltd.) | 0.24 |
| Meiji Chocolate Kouka cacao 86% (manufactured by Meiji Co., Ltd.) | 0.25 |
| Carré de chocolat < Cacao 70 > (manufactured by Morinaga & Co., Ltd.) | 0.19 |

[Table 4]

| Sample | GABA amount | Sphingolipid amount |
|---|---|---|
| Essence of cacao bean-ground product | 0.27 mg/mL | - |
| Sweet chocolate using cacao bean-ground product | 0.64 mg/g | 0.32mg/g |
| Milk chocolate using cacao bean-ground product | 0.38 mg/g | 0.19mg/g |

The essence abundantly contained GABA derived from cacao beans, but had a good flavor. The chocolates abundantly contained GABA derived from cacao beans, but had the same flavor and food texture as normal sweet chocolate or milk chocolate.

(Specific gravity)

[Table 5]

| Raw material: Cacao bean A | Ground product a | Ground product b | Ground product c | Ground product d | Ground product e |
|---|---|---|---|---|---|
| Specific gravity | 1.0 | 1.1 | 1.2 | 1.3 | 1.4 |
| GABA (mg/g) | 0.45 | 0.54 | 0.86 | 1.13 | 1.16 |
| Sphingolipid (mg/g) | 0.25 | 0.25 | 0.42 | 0.50 | 0.52 |

Using a cacao bean B different from the cacao bean A in roasting method and a cacao bean C different from the cacao bean A in production area, GABA and sphingolipid of the cacao-ground products were measured for each specific gravity.

The measurement results of GABA are shown in Table 6. The section with a specific gravity of 1.4 of the cacao bean B was not contained in the ground product and could not be fractionated, but it was found that when both the cacao beans B and C had a specific gravity of 1.2 or more, they contained 0.55 mg/g or more of GABA.

[Table 6]

| GABA (mg/g) | | | | | |
|---|---|---|---|---|---|
| | Specific gravity 1.0 | Specific gravity 1.1 | Specific gravity 1.2 | Specific gravity 1.3 | Specific gravity 1.4 |
| Cacao bean B | 0.37 | 0.24 | 0.78 | 0.93 | - |
| Cacao bean C | 0.44 | 0.46 | 0.72 | 0.86 | 0.63 |

The measurement results of sphingolipid are shown in Table 7. The section with a specific gravity of 1.4 of the cacao bean B was not contained in the ground product and could not be fractionated, but it was found that when both the cacao beans B and C had a specific gravity of 1.2 or more, they contained 0.26 mg/g or more of a sphingolipid.

[Table 7]

| Sphingolipid (mg/g) | | | | | |
|---|---|---|---|---|---|
| | Specific gravity 1.0 | Specific gravity 1.1 | Specific gravity 1.2 | Specific gravity 1.3 | Specific gravity 1.4 |
| Cacao bean B | 0.36 | 0.39 | 0.58 | 0.61 | - |
| Cacao bean C | 0.34 | 0.35 | 0.57 | 0.64 | 0.5 |

<Comparison of sphingolipid with conventional product and the like>

In addition, the measurement results of the sphingolipid content in conventional products and the like are shown in the following table.

[Table 8]

| | Sphingolipid (mg/g) |
|---|---|
| Cacao mass A | 0.19 |
| Cacao mass B | 0.22 |

(continued)

|  | Sphingolipid (mg/g) |
|---|---|
| Cocoa A | 0.20 |
| Cocoa B | 0.42 |
| Chocolate Kouka cacao 72% | 0.20 |
| Chocolate Kouka cacao 82% | 0.24 |
| Dried product of neutral cacao essence | 0.07 |
| Residue of neutral cacao extraction (moisture 66%) | 0.12 |
| Cocoa butter | 0 |
| Cocoa A: a cocoa powder having an oil content of 12%, obtained by squeezing cacao mass obtained by grinding cacao beans that have not been subjected to alkali treatment<br>Cocoa B: a cocoa powder having an oil content of 12%, obtained by squeezing cacao mass obtained by grinding alkali-treated cacao beans<br>Dried product of neutral cacao essence: fermented cacao beans → cacao nib → extraction → freeze-dried<br>Residue of neutral cacao extraction: cacao mass A → extracted with hot water at 85 °C → centrifugation → precipitation part (extraction residue) (moisture 66%) | |

Cocoa is obtained by squeezing cocoa butter from cacao mass to reduce the oil content, but it is considered that the sphingolipid becomes relatively high by removing the oil content.

<Oils and fats formulation>

(Triglycerides)

The analysis results of the triglyceride formulation are shown in the following table.

[Table 9]

|  | Sample 27 | | Sample 24 | |
|---|---|---|---|---|
|  | Composition 1 | Cacao nib 1 | Composition 2 | Cacao nib 2 |
| Triglyceride | Formulation (%) | | Formulation (%) | |
| LnLnL | - | - | 0.2 | - |
| LLnL | - | - | 0.4 | - |
| LnLP | - | - | 2.4 | - |
| LLL | 1.6 | - | 1.9 | - |
| LLO | 2.5 | - | 1.7 | - |
| PLL | 6.0 | - | 8.3 | - |
| OLO | 0.6 | - | 1.1 | 0.1 |
| PLO | 5.5 | 0.5 | 6.9 | 0.9 |
| PLP | 8.7 | 1.9 | 12.3 | 2.9 |
| OOO | 0.7 | 0.3 | 1.1 | 0.4 |
| POO | 4.4 | 2.4 | 5.6 | 3.7 |
| POP | 13.9 | 19.0 | 14.5 | 23.2 |
| PPP | 1.0 | 0.3 | - | - |
| SOO | 15.3 | 2.7 | 12.3 | 3.4 |
| SOP | 12.9 | 39.6 | 12.2 | 39.9 |

(continued)

| Triglyceride | Sample 27 | | Sample 24 | |
|---|---|---|---|---|
| | Composition 1 | Cacao nib 1 | Composition 2 | Cacao nib 2 |
| | Formulation (%) | | Formulation (%) | |
| SPP | 0.6 | 0.6 | 0.6 | 0.5 |
| OOA | - | - | 0.7 | 0.1 |
| SOS | 11.0 | 28.7 | 7.5 | 21.6 |
| SSP | 3.1 | 0.8 | 0.2 | 0.6 |
| SOA | 1.2 | 1.6 | 0.9 | 1.1 |
| SSS | 0.8 | 0.4 | - | 0.2 |
| Others | 10.2 | 1.2 | 9.2 | 1.4 |
| Total | 100.0 | 100.0 | 100.0 | 100.0 |
| P: Palmitic acid residue, S: Stearic acid residue, O: Oleic acid residue, L: Linoleic acid residue, Ln: Linolenic acid residue, and A: Arachidic acid residue. "-" represents the measurement limit or less (less than 0.1). | | | | |

The compositions 1 and 2 contain LLL, LLO, and PLL, which are not contained in the cacao nib 1 and 2, as a triglyceride whose β-position is linoleic acid. The total triglycerides in the composition 1 contain LLL, LLO, and PLL in a sum total amount of 10.1%, and the total triglycerides in the composition 2 contain them in a sum total amount of 11.9%. The total triglycerides in the composition 1 contain LLL, LLO, PLL, OLO, PLO, and PLP in a sum total amount of 24.9%, and the total triglycerides in the composition 2 contain them in a sum total amount of 32.2%. On the other hand, although the cacao nibs contained OLO, PLO, and PLP, their total amount was 2.4% for the cacao nib 1 and 3.9% for the cacao nib 2. In the composition of the present invention, the total triglycerides in the composition 1 contain OLO, PLO, and PLP in a sum total amount of 14.8%, and the total triglycerides in the composition 2 contain them in a sum total amount of 20.3%.

The total triglycerides in the cacao nib 1 contain SOP in an amount of 39.6%, and SOS in an amount of 28.7%, which were high. The total triglycerides in the cacao nib 2 contain SOP in an amount of 39.9%, and SOS in an amount of 21.6%, which were high. On the other hand, the total triglycerides in the compositions 1 contain SOP in an amount of 12.9%, and SOS in an amount of 11.0%. The total triglycerides in the compositions 2 contain SOP in an amount of 12.2%, and SOS in an amount of 7.5%, which mean they were less than 20%, respectively.

The cacao bean-derived compositions of the present invention, having a configuration clearly different from the triglyceride formulation contained in cacao nib, can be expected to exhibit functional characteristics different from those of conventional processed products of cacao beans also containing cacao mass obtained by grinding cacao nib. In addition, the cacao bean-derived composition of the present invention can be used not only as a source of GABA derived from cacao beans but also as a source of linoleic acid.

(Fatty acid formulation)

The fatty acid formulations of the cacao bean-derived composition and the cacao nibs are shown in the following table. For comparison, the fatty acid formulations of common palm oil, palm kernel oil, coconut oil, and sunflower oil (high linoleic product and high oleic product) are shown in the following table. Values described on the web page of KANEDA Co., Ltd (https://www.kaneda.co.jp/jigyou/oils_composition.html), which are referred to from Japan Inspection Institute of Fats & Oils, are used.

[Table 10]

| Fatty acid | | Formulation (%) | |
|---|---|---|---|
| Name | Number of carbon atoms / Number of double bonds | Composition 1 (Sample 27) | Composition 2 (Sample 24) |
| Capric acid | C10:0 | 0.2 | 0.2 |
| Lauric acid | C12:0 | 0.5 | 0.4 |

(continued)

| Fatty acid | | Formulation (%) | |
|---|---|---|---|
| Myristic acid | C14:0 | 0.4 | 0.5 |
| Pentadecanoic acid | C15:0 | 0.2 | 0.1 |
| Palmitic acid | C16:0 | 30.9 | 31.6 |
| Palmitoleic acid | C16:1 | 0.6 | 0.5 |
| Heptadecanoic acid | C17:0 | 0.7 | 0.6 |
| Stearic acid | C18:0 | 13.7 | 10.5 |
| Oleic acid | C18:1 | 15 | 14.5 |
| Linoleic acid | C18:2 | 32.7 | 36.0 |
| Linolenic acid | C18:3 | 2.3 | 2.4 |
| Arachidic acid | C20:0 | 1.3 | 1.1 |
| Behenic acid | C22:0 | 0.8 | 0.8 |
| Lignoceric acid | C24:0 | 0.7 | 0.8 |
| Total | | 100 | 100 |

[Table 11]

| Fatty acid | | Formulation (%) | | | | | | |
|---|---|---|---|---|---|---|---|---|
| Name | Number of carbon atoms/ Number of double bonds | Cacao nib 1 (Sample 27) | Cacao nib 2 (Sample 24) | Palm oil | Palm kernel oil | Coconut oil | Sunflower oil High linoleic | Sunflower oil High oleic |
| Caprylic acid | C8:0 | - | - | - | 2.7 | 5.9 | - | - |
| Capric acid | C10:0 | - | - | - | 2.9 | 5.3 | - | - |
| Lauric acid | C12:0 | - | - | 0.3 | 46.8 | 47.7 | - | - |
| Myristic acid | C14:0 | - | - | 1.0 | 16.3 | 19.0 | 0.0 | 0.0 |
| Pentadecanoic acid | C15:0 | - | - | - | - | - | - | - |
| Palmitic acid | C16:0 | 24.7 | 28.8 | 44.6 | 9 | 9.7 | 6.3 | 3.7 |
| Palmitoleic acid | C16:1 | 0.3 | 0.3 | 0.2 | 0.0 | - | 0.1 | 0.1 |
| Heptadecanoic acid | C17:0 | 0.2 | 0.3 | - | - | - | - | - |
| Stearic acid | C18:0 | 37.3 | 32.0 | 4.3 | 2.6 | 3.2 | 3.7 | 2.8 |
| Oleic acid | C18:1 | 32.8 | 33.2 | 39.4 | 16.7 | 7.1 | 30.8 | 83.7 |
| Linoleic acid | C18:2 | 3.2 | 4.1 | 9.4 | 2.6 | 1.7 | 57.6 | 7.8 |
| Linolenic acid | C18:3 | 0.2 | 0.2 | 0.1 | - | 0.0 | 0.1 | 0.2 |
| Arachidic acid | C20:0 | 1.1 | 0.9 | 0.4 | 0.1 | 0.1 | 0.3 | 0.3 |
| Eicosenoic acid | C20:1 | - | - | 0.1 | 0.1 | 0.0 | 0.2 | 0.3 |
| Behenic acid | C22:0 | 0.2 | 0.2 | 0.0 | 0.0 | - | 0.7 | 0.9 |
| Lignoceric acid | C24:0 | - | - | 0.0 | 0.0 | 0.0 | 0.3 | 0.3 |

(continued)

| Fatty acid | | Formulation (%) | | | | | | |
|---|---|---|---|---|---|---|---|---|
| Name | Number of carbon atoms/ Number of double bonds | Cacao nib 1 (Sample 27) | Cacao nib 2 (Sample 24) | Palm oil | Palm kernel oil | Coconut oil | Sunflower oil High linoleic | Sunflower oil High oleic |
| Total | | 100 | 100 | 100 | 100 | 100 | 100 | 100 |

The total fatty acids in the composition 1 contain palmitic acid, stearic acid, and oleic acid in a sum total amount of 59.6%, and the total fatty acids in the composition 2 contain them in a sum total amount of 56.6%. The total fatty acids in cacao nib 1 contain palmitic acid, stearic acid, and oleic acid in a sum total amount of 94.8%, and the total fatty acids in cacao nib 2 contain them in a sum total amount of 94.0%. In addition, the total fatty acids in the composition 1 contain linoleic acid in a large amount of 32.7%, and the total fatty acids in the composition 2 contain linoleic acid in a large amount of 36.0%. However, the total fatty acids in the cacao nib 1 contain linoleic acid in a small amount of 3.2%, and the total fatty acids in the cacao nib 2 contain linoleic acid in a small amount of 4.1%. The compositions 1 and 2 contained capric acid, lauric acid, myristic acid, and pentadecanoic acid, which were not contained in the cacao nibs, and the total fatty acids in the composition 1 contain them in a sum total amount of 1.3% and the total fatty acids in the composition 2 contain them in a sum total amount of 1.2%. In addition, the total fatty acids in the composition 1 contain palmitic acid in a relatively large amount of 30.9%, and the total fatty acids in the composition 2 contain palmitic acid in a relatively large amount of 31.6%.

The lipid content of each sample was 3.9 g/100 g for the composition 1, 5.1 g/100 g for the composition 2, 55.9 g/100 g for the cacao nib 1, and 52.8 g/100 g for the cacao nib 2.

Since the cacao bean-derived composition of the present invention is clearly different from conventional cacao nib in fatty acid formulation, it can be expected that the cacao bean-derived composition exhibits functional characteristics different from those of conventional processed products of cacao beans, which also include cacao mass obtained by grinding cacao nib. In addition, the cacao bean-derived composition of the present invention can be used not only as a source of GABA derived from cacao beans but also as a source of linoleic acid.

[Production Example 5: Cacao bean-derived composition (germ)]

The cacao bean of Sample 3 in Table 2 is cut with a cutter, and the germ portion (embryo bud) is collected. Since the germ portion is clearly different in color and hardness from other surrounding tissues, it can be easily recognized. The collected germ portion had a specific gravity of 1.2 or more.

<GABA content and sphingolipid content in germ portion of cacao bean>

The results of measuring the content of GABA and sphingolipid in the germ portion are shown in the following table. The measurement was performed by the method described in the section of [Measurement method].

[Table 12]

| Raw material | Germ |
|---|---|
| Specific gravity | 1.2 |
| GABA (mg/g) | 0.86 |
| Sphingolipid (mg/g) | 0.42 |

The germ portion of cacao corresponds to the cacao bean-derived composition of the present invention, and contained GABA and sphingolipid at a high ratio.

[Production Example of Food]

<Drink powder>

(1) 34.5 g of the composition of the present invention is ground to about 200 $\mu$m with a mill.
(2) To (1), 17.9 g of cocoa butter, 18.0 g of powdered sugar, 19.0 g of whole milk powder, and 0.6 g of lecithin are added and mixed, and then ground with a refiner to form flakes.

The drink powder containing the composition of the present invention (Sample 22) contained 28 mg of GABA in 58 g. For example, by dissolving 58 g of the powder in water so as to be 200 mL, and taking the whole amount, functionality of stress relaxation and fatigue reduction can be expected. In addition, a beverage obtained from the drink powder containing the product of the present invention has a good flavor. The drink powder contains 0.22 mg/g of cacao ceramide.

<Chocolate>

(1) 5 g of the composition of the present invention is ground to about 30 $\mu$m with a roll.
(2) (1) was mixed with 60 g of cacao mass, 10 g of powdered sugar, and 0.03 g of lecithin.
(3) (2) is tempered, then poured into a mold, and cooled.

<Yokan>

(1) The composition of the present invention is ground to about 30 $\mu$m with a roll.
(2) Water and agar are combined and heated in a pot until boiling.
(3) After the mixture is boiled and the agar is completely dissolved, sugar and brown sugar are added and melted by boiling.
(4) (1) was added to and uniformly mixed with (2), and heated until boiling.
(5) The mixture is removed from heat, then poured into a mold, and cooled.

<Paste>

(1) The composition of the present invention is ground to about 30 $\mu$m with a roll.
(2) Powdered sugar is added to (1) and mixed uniformly, and water is added until a moist paste is obtained.

<Spread>

(1) The composition of the present invention is ground to about 30 $\mu$m with a roll.
(2) Water is boiled and granulated sugar, brown sugar, and salt are dissolved.
(3) (1) was added to and uniformly mixed with (2), and heated until boiling.
(4) Water and agar are put in a pot in combination, and heated until boiling.
(5) After (4) is boiled and the agar is completely dissolved, (3) is added and uniformly mixed, and the mixture is heated until boiling.
(6) Salt, salt-free butter, vanillin, and sweetened condensed milk are put in a ball, and (5) is added and mixed.
(7) The mixture is poured into a mold, cooled down, and then cooled in a refrigerator.

<Pasta>

(1) The composition of the present invention is ground to about 30 μm with a roll.

(2) Strong flour, olive oil, egg, and salt are added to (1), and mixed as if the mixture is cut.

(3) After the dough is formed into a crumbled shape, the dough is kneaded into a lump by hand.

(4) After the dough is further kneaded to have a hardness of about an earlobe, the dough is divided into halves, rolled into a ball, and wrapped in plastic wrap, and the dough is rested for 15 minutes or more.

(5) The dough is dusted and stretched, and cut into a noodle shape.

(6) The dough is boiled with boiling water.

<Smoothie>

(1) The composition of the present invention is ground to about 30 μm with a roll.

(2) Granulated sugar, salt, and an emulsifier are added to water and dissolved.

(3) (1) is added to (2), and mixed until uniform.

<Ice>

(1) The composition of the present invention is ground to about 30 μm with a roll.

(2) Water, dairy products, and vegetable oils and fats are stirred while being heated.

(3) After reaching 40 to 50 °C, sugar, an emulsifier, and a stabilizer are added.

(4) The mixture is holed at 75°C for 10 minutes.

(5) After completion of heating, homogenization is performed by a shearing force.

(6) (1) is added to and mixed with the homogenized ice cream mix, and then cooled.

(7) Freezing is performed using a gelato machine manufactured by CARPIGIANI.

<Gummy>

(1) The composition of the present invention is ground to about 30 μm with a roll.

(2) Sugar, starch syrup, polydextrose, sorbitol, and glycerin are boiled down to 125°C.

(3) When the product temperature decreases to 110°C, gelatin, a gelling agent, and an emulsifier are added and mixed.

(4) An acidulant is added and mixed.

(5) (1) is added and mixed.

(6) Water is added to adjust Bx to 79.5.

(7) The mixture is deposited to corn starch and aged.

(8) The resulting gummy is demolded after 24 hours.

<Jelly>

(1) The composition of the present invention is ground to about 30 μm with a roll.

(2) Gelatin is soaked in water.

(3) Milk is warmed until just before boiling, (1) is added thereto and stirred, and then (2) is added thereto.

(4) (3) is put in cold water to cool down, and fresh cream is added and stirred until thickened.

(5) The mixture is poured into a mold, and cooled and solidified in a refrigerator.

<Tablet confectionery>

(1) 5 g of the composition of the present invention is ground to about 30 μm with a roll.
(2) To (1), 5 g of powdered sugar is added and mixed.
(3) 0.3 g of citric acid, 0.6 g of lemon juice, and 0.6 g of water are added and mixed.
(4) (3) is added to (2), and mixed until there are no lumps.
(5) (4) is added with 0.5 g of sodium bicarbonate and mixed.
(6) (5) is put in a tray, covered with a wrap, and pressed with a finger.
(7) (6) is peeled off, placed on a tray, and dried in an oven at 70 to 80°C for about 1 hour.

A tablet confection containing the composition of the present invention (sample 27) contains 0.5 mg of GABA in one tablet (1 g).

**Claims**

1. A cacao bean-derived composition having a specific gravity of 1.2 or more and containing 0.55 mg/g or more of γ-aminobutyric acid (GABA).

2. A cacao bean-derived composition containing at least one triglyceride selected from the group consisting of LLL, LLO, PLL, and OLO; and 0.55 mg/g or more of GABA, wherein L represents a linoleic acid residue, O represents an oleic acid residue, and P represents a palmitic acid residue.

3. The composition according to claim 1 or 2, wherein total triglycerides in the composition contain LLL, LLO, PLL, and OLO in a sum total amount of 1% or more.

4. The composition according to claim 1 or 2, wherein total triglycerides in the composition contain LLL, LLO, PLL, OLO, PLO, and PLP in a sum total amount of 5% or more.

5. The composition according to claim 1 or 2, wherein total triglycerides in the composition contain PLO and PLP in a sum total amount of 5% or more.

6. The composition according to any one of claims 1 to 5, wherein total triglycerides in the composition contain at least one selected from the group consisting of SOP and SOS in an amount of less than 20%, wherein S represents a stearic acid residue.

7. A cacao bean-derived composition,

   having a fatty acid formulation which comprises 1) to 4):

   1) total fatty acids in the composition contain palmitic acid, stearic acid, and oleic acid in a sum total amount of less than 90%;
   2) total fatty acids in the composition contain linoleic acid in an amount of 20 to 40%;
   3) total fatty acids in the composition contain capric acid, lauric acid, myristic acid, and pentadecanoic acid in a sum total amount of 0.1 to 5%; and
   4) total fatty acids in the composition contain palmitic acid in an amount of 20% or more, and

   containing 0.55 mg/g or more of GABA.

8. A processed product of the composition according to any one of claims 1 to 7.

9. A food containing the composition according to any one of claims 1 to 7.

10. A method for producing a composition containing GABA, the method comprising:
    obtaining a fraction having a specific gravity of 1.2 or more from a cacao bean-ground product.

11. A method for producing a food, the method comprising:
producing a composition by the method according to claim 10, and adding the produced composition to a food.

12. A method for producing a food, the method comprising:
adding a processed product of cacao beans to a food, wherein the processed product of cacao beans is a cacao bean-ground product having a specific gravity of 1.2 or more, and thereby a cacao-derived GABA is reinforced in the food.

## INTERNATIONAL SEARCH REPORT

| International application No. |
|---|
| **PCT/JP2022/036734** |

### A. CLASSIFICATION OF SUBJECT MATTER

*A23L 33/105*(2016.01)i; *A23G 1/06*(2006.01)i; *A23G 1/32*(2006.01)i; *A23G 1/36*(2006.01)i; *A23G 1/48*(2006.01)i
FI: A23L33/105; A23G1/36; A23G1/32; A23G1/48; A23G1/06

According to International Patent Classification (IPC) or to both national classification and IPC

### B. FIELDS SEARCHED

Minimum documentation searched (classification system followed by classification symbols)

A23L33/105; A23G1/06; A23G1/32; A23G1/36; A23G1/48

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | カカオ, チョコレートの話 [online], 13 August 2020 [retrieval date 21 November 2022], internet:<URL: https://chocolumbus.com/column/cacao/entry-126.html>  in particular, terrine chocolat, non-official translation (The story of cacao and chocolate [online]) | 1-12 |
| A | チョコレートワールド [online], 07 August 2020 [retrieval date 21 November 2022], internet:<URL: https://chocolateworld123.com/cacaobeantochocolate/>  in particular, Sorting, non-official translation (Chocolate world [online]) | 1-12 |
| A | 食品添加物と健康食品の真実 [online], 27 July 2019 [retrieval date 21 November 2022], internet:<URL: https://syokunokaisetu.com/chocolate2/>  in particular, Sorting (cleaner), non-official translation (The truth about food additives and healthy foods [online]) | 1-12 |
| A | 中西 喜次, カカオ脂とカカオ代用脂, 油化学, 1970, vol. 19, no. 8, pp. 722-733  in particular, general properties of cocoa butter, (NAKANISHI, Yoshitsugu. Cacao Butter and Substituted Cacao Butter. Journal of Japan Oil Chemists' Society.) | 1-12 |

☑ Further documents are listed in the continuation of Box C.   ☑ See patent family annex.

| | |
|---|---|
| *      Special categories of cited documents:  "A"   document defining the general state of the art which is not considered to be of particular relevance  "E"   earlier application or patent but published on or after the international filing date  "L"   document which may throw doubts on priority claim(s) or which is cited to establish the publication date of another citation or other special reason (as specified)  "O"   document referring to an oral disclosure, use, exhibition or other means  "P"   document published prior to the international filing date but later than the priority date claimed | "T"   later document published after the international filing date or priority date and not in conflict with the application but cited to understand the principle or theory underlying the invention  "X"   document of particular relevance; the claimed invention cannot be considered novel or cannot be considered to involve an inventive step when the document is taken alone  "Y"   document of particular relevance; the claimed invention cannot be considered to involve an inventive step when the document is combined with one or more other such documents, such combination being obvious to a person skilled in the art  "&"   document member of the same patent family |

| Date of the actual completion of the international search | Date of mailing of the international search report |
|---|---|
| **21 November 2022** | **13 December 2022** |

| Name and mailing address of the ISA/JP | Authorized officer |
|---|---|
| **Japan Patent Office (ISA/JP)**  **3-4-3 Kasumigaseki, Chiyoda-ku, Tokyo 100-8915**  **Japan** | |
| | Telephone No. |

Form PCT/ISA/210 (second sheet) (January 2015)

**INTERNATIONAL SEARCH REPORT**

| International application No. |
|---|
| **PCT/JP2022/036734** |

### C. DOCUMENTS CONSIDERED TO BE RELEVANT

| Category* | Citation of document, with indication, where appropriate, of the relevant passages | Relevant to claim No. |
|---|---|---|
| A | macaroni [online], 26 August 2020 [retrieval date 21 November 2022], internet:<URL: https://macaro-ni.jp/91288><br>entire text | 1-12 |
| A | JP 2007-006853 A (EZAKI GLICO CO LTD) 18 January 2007 (2007-01-18) | 1-12 |
| A | JP 9-075003 A (MORINAGA & CO LTD) 25 March 1997 (1997-03-25) | 1-12 |

Form PCT/ISA/210 (second sheet) (January 2015)

## INTERNATIONAL SEARCH REPORT

| International application No. |
| --- |
| **PCT/JP2022/036734** |

**Box No. III    Observations where unity of invention is lacking (Continuation of item 3 of first sheet)**

This International Searching Authority found multiple inventions in this international application, as follows:

Since the GABA-containing cacao bean-derived composition, which is the feature shared by the inventions in claims 1, 2, 7, 10, and 12, does not make a contribution over the prior art in light of the disclosure of document 1, said technical feature cannot be said to be a special technical feature.

Therefore, there are no same or corresponding special technical features among the inventions in claims 1, 2, 7, 10, and 12.

1. ☐  As all required additional search fees were timely paid by the applicant, this international search report covers all searchable claims.

2. ☑  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on Protest**    ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet) (January 2015)

INTERNATIONAL SEARCH REPORT
Information on patent family members

International application No.

**PCT/JP2022/036734**

| Patent document cited in search report | Publication date (day/month/year) | Patent family member(s) | Publication date (day/month/year) |
|---|---|---|---|
| JP 2007-006853 A | 18 January 2007 | (Family: none) | |
| JP 9-075003 A | 25 March 1997 | (Family: none) | |

Form PCT/ISA/210 (patent family annex) (January 2015)

# EP 4 410 116 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009201383 A
- JP 2005013127 A
- JP 4041020 B
- JP 2005348656 A
- JP 2007006853 A
- JP 2005295995 A
- JP 4359205 B
- US 20010041683 A1

### Non-patent literature cited in the description

- **ANGELA MARSEGLIA ; GERARDO PALLA ; AUGUSTA CALIGIANI.** Presence and variation of γ-aminobutyric acid and other free amino acids in cocoa beans from different geographical origins. *Food Research International,* 2014, vol. 63, 360-366
- **YOSHITSUGU NAKANISHI.** Cacao Butter and Substituted Cacao Butter. *Oil Chemistry,* 1970, vol. 19 (8), 722-733